# EUROPEAN PATENT APPLICATION

(11) **EP 3 622 881 A1**
(43) Date of publication of application: **18.03.2020**
(21) Application number: 18194472.9
(22) Date of filing: 14.09.2018
(51) Int. Cl.: A61B 5/00

(54) **METHOD AND APPARATUS FOR HUMAN SENSORY TESTING**

(71) Applicant: Nokia Technologies Oy, 02610 Espoo (FI)
(72) Inventor: Müller, Kiti, 00430 Helsinki (FI); Rajala, Satu, 36200 Kangasala (FI)
(74) Representative: Espatent Oy

(57) **Abstract**

An apparatus for human sensory testing, comprising imaging means for imaging a target area; at least one touch tool configured to touch a location on the target area; and control means; wherein the imaging means are configured to image the location touched on the target area; and wherein the control means are configured to send the image and/or information on the location and to receive information regarding user input.

## Description

### TECHNICAL FIELD

The present application generally relates to a method and system for sensory testing. In particular, but not exclusively, the present application relates to a method and system for cognitive and sensory testing.

### BACKGROUND

This section illustrates useful background information without admission of any technique described herein representative of the state of the art.

Humans gather information from their surroundings via different senses. This information is then relayed by sensory nerves to the brain where it is processed. Currently, cognitive and memory testing based on sensing is carried out using visual or auditory tests. Sometimes human ability to identify forms by touch sense is tested by drawing forms on skin manually, or the subject is asked to identify an object placed on the palm of the hand.

The current invention provides a system and method for testing cognitive capabilities and processes, for example receptor sensing capabilities, functions of the different cortices of the brain, working or long-term memory, attention and their interaction by utilizing skin and/or tongue surfaces.

### SUMMARY

Various aspects of examples of the invention are set out in the claims.

According to a first example aspect of the present invention, there is provided an apparatus for human sensory testing, comprising
imaging means for imaging a target area;
at least one touch tool configured to touch a location on the target area; and control means; wherein
the imaging means are configured to image the location touched on the target area; and wherein the control means are configured to send the image and/or information on the location and to receive information regarding user input.

The apparatus may further comprise measurement means configured to measure a force or pressure with which the at least one touch tool touches the location on the target area.

The apparatus may further comprise actuating means for providing a stimulus or stimuli causing a sensation on the location on the target area which the at least one touch tool is touching.

The apparatus may comprise a plurality of touch tools, each touch tool having a different shape and/or surface texture.

According to a second example aspect of the present invention, there is provided a system for sensory testing comprising
a first apparatus according to the first example aspect of the invention; and
a second apparatus connected with the first apparatus, and comprising a processor and a user interface unit.

The user interface unit is configured to receive user input.

The user interface unit comprises a touch display.

The second apparatus may comprise a personal electronic device.

According to a third example aspect of the present invention, there is provided a method for human sensory testing, comprising
imaging a target area to be used for sensory testing with imaging means of a first apparatus;
touching a location on the target area with at least one touch tool of a first apparatus;
imaging the location touched on the target area and sending the image and/or information on the location to a second apparatus; and
calculating the location from the image of the touched location and/or using the information on the location.

The method may further comprise measuring the force or pressure with which the at least one touch tool is touching the location on the target area.

The method may further comprise providing a stimulus or stimuli causing a sensation on the location on the target area.

The method may further comprise receiving user input with a user input unit of the second apparatus.

Receiving user input may comprise the user indicating the location on the target area from an image on the user interface unit.

The target area may comprise human tongue or an area of human skin.

Any foregoing memory medium may comprise a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1 shows a schematic view of a system according to an example embodiment of the present invention;
Fig. 2 shows a block diagram of a system according to an example embodiment of the present invention; and
Fig. 3 shows a flow chart of a method according to an example embodiment of the present invention.

### DETAILED DESCRIPTON OF THE DRAWINGS

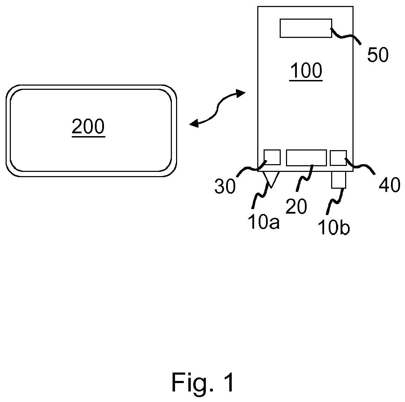

An example embodiment of the present invention and its potential advantages are understood by referring to Figs. 1 through 3 of the drawings. In this document, like reference signs denote like parts or steps.

Fig. 1 shows a schematic view of a system according to an example embodiment of the present invention. The system comprises a first apparatus 100 comprising a testing element and a second apparatus 200 comprising a user interface element. In an embodiment, the first apparatus 100 and the second apparatus 200 are comprised in a single apparatus. In an embodiment, the first apparatus 100 and the second apparatus 200 are connected with a wired or a wireless connection.

In an example embodiment, the connection between the first 100 and second 200 apparatus, as well as between the elements of the first 100 and/or second 200 apparatus described hereinafter comprises a connection using, for example, a local area network (LAN), a wireless local area network (WLAN), Bluetooth, near field communication (NFC), cellular data communication, or satellite data communication. In an example embodiment, the second apparatus 200 comprises or is comprised in an external device, such as a personal electronic device such as a smartphone, a smartwatch, a tablet computer, a laptop computer, a desktop computer, laboratory equipment, or a cloud service.

The first apparatus 100 comprises at least one touch tool 10a,10b for touching, i.e. applying pressure to, a target location comprising a location on the surface of human skin or human tongue. In an embodiment, the first apparatus 100 comprises a plurality of touch tools 10a,10b, each touch tool having a different tip shape and/or tip surface texture. In an example embodiment, the first apparatus comprises a sharp tipped touch tool 10a and a blunt tipped touch tool 10b. In an example embodiment, the first apparatus comprises a touch tool 10,10b with a smooth surface and a touch tool 10a,10b with a rough surface.

In an example embodiment, the first apparatus comprises a plurality of touch tool 10a,10b with varying surface sharpness and/or surface roughness and/or material and/or hardness. In an example embodiment, the at least one touch tool 10a,10b comprises an arrangement for varying the physical properties thereof for example an arrangement for providing a varying temperature or moistness. In a further example embodiment, the at least one touch tool 10a,10b comprises an arrangement for providing a taste that can be perceived by the tongue surface touched therewith.

In an example embodiment, the at least one touch tool 10a,10b is formed as an integral part of the first apparatus 100. In a further example embodiment, the at least one touch tool 10a,10b is detachable and attachable from and to the first apparatus 100. In such a case the at least one touch tool 10a,10b, in an example embodiment is connected with the first apparatus with a wired or wireless connection.

The first apparatus 100 further comprises imaging means 20. In an example embodiment, the imaging means 20 is configured to image a target, such as a location on a surface which is touched with the at least one touch tool 10,10b. In an example embodiment, the imaging means comprise an imaging unit comprising elements such as a charge coupled device, CCD, imaging sensor, a complementary metal oxide semiconductor, CMOS, imaging sensor, a photometric stereo imaging system, or a further digital imaging system. In an example embodiment, the imaging means 20 is integrated with the first apparatus 100. In a further example embodiment, the imaging means 20 is integrated with the second apparatus 200. In a still further example embodiment, the imaging means comprises a separate device in communication with the first apparatus 100 and/or the second apparatus 200.

The first apparatus 100 further comprises, in an example embodiment, measurement means 30. In an example embodiment, the measurement means 30 are integrated with the at least one touch tool 10a,10b. The measurement means 30 are configured to measure a force or pressure with which the touch tool is pushed or applied to the target, i.e. to the surface which is touched with the at least one touch tool 10a,10b. In an example embodiment, the measurement means comprise a force sensor and/or a pressure sensor. In an example embodiment, the first apparatus further comprises illuminating means for illuminating the target. In a further example embodiment, the illuminating means are used to illuminate the locations to be touched, for example in accordance with a previously stored pattern of locations or a predetermined pattern of locations. In an example embodiment, the illumination is used to guide the testing and the person carrying out the testing, i.e. to ascertain that a certain pattern of locations is touched during testing.

The first apparatus 100 further comprises, in an example embodiment, actuator means 40. In an example embodiment, the actuator means 40 are integrated with the at least one touch tool 10a,10b. The actuator means 40 are configured to provide a stimulus or stimuli causing a sensation at the target is touched with the at least one touch tool 10a,10b. In example embodiment, the stimulus comprises stimuli such as vibration or a force impulse causing a tactile sensation. In a further example embodiment, the stimulus comprises a change of temperature such as heating or cooling. In a further example embodiment, the stimulus comprises providing a component having a recognizable taste.

The first apparatus 100 further comprises, in an example embodiment, control means 50. The control means 50 are configured to control the functions of the elements of the first apparatus 100 and to communicate with the second apparatus 200.

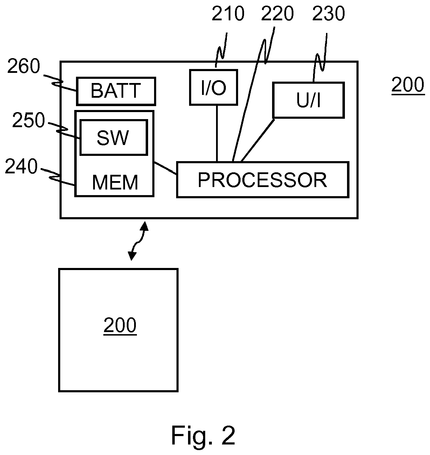

Fig. 2 shows a block diagram of the system according to an example embodiment of the invention with a first 100 and a second 200 apparatus. The second apparatus 200 comprises a memory 240 including computer program code 250. In an example embodiment, the memory 240 comprises a memory medium comprising a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

The second apparatus 200 further comprises a processor 220 for controlling the operation of an apparatus using the computer program code 250, a communication unit 210 for communicating with other apparatuses and/or with the elements of the first apparatus 100, for example with the at least one touch tool 10a,10b. The second apparatus 200 further comprises a user interface unit 230. The user interface unit is configured to receive user input e.g. from the subject to be tested. In an example embodiment, the user interface unit 230 comprises a touch display or a display and buttons. In an example embodiment, the user interface unit comprises, on the outer surface of the second apparatus 200 elements for controlling the functions of the second apparatus 200. The second apparatus 200 further comprises, in an example embodiment, a battery 260.

In an example embodiment, the communication unit 210 is configured to provide communication with the first apparatus 100 and/or with the elements of the first apparatus, as well as with any external apparatus such as a smartphone, a smartwatch, wristband, ankleband or armband a tablet computer, a laptop computer, a desktop computer, laboratory equipment, or a cloud service. The communication unit 210 comprises, for example, a local area network (LAN) port, a wireless local area network (WLAN) unit, Bluetooth unit, cellular data communication unit, or satellite data communication unit.

The processor 220 comprises, for example, any one or more of: a master control unit (MCU), a microprocessor, a digital signal processor (DSP), an application specific integrated circuit (ASIC), a field programmable gate array, and a microcontroller. In a further example embodiment, the second apparatus 200, or a part of it is comprised in an external apparatus or in a cloud service.

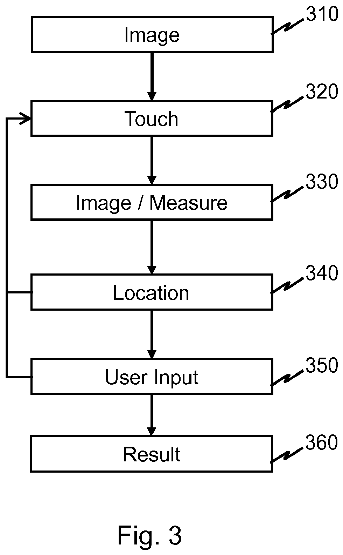

Fig. 3 shows a flow chart of a method according to an example embodiment. It is to be noted that the processor 220 and/or control means 50 are configured to cause carrying out the steps described hereinafter. At step 310 the imaging means 20 are used to obtain a general image of the target area that is to be used for sensory testing according to an embodiment of the invention. In an example embodiment the target area comprises a human tongue or a certain area of skin, such as a palm of a hand. In an example embodiment, the obtained image, a still image or video image, is sent to the second apparatus 200 for processing and/or for displaying on the user interface unit.

At step 320 a location on the target area is touched with a touch tool 10a,10b. In an embodiment, the type of touch tool is chosen in accordance with the desired test or test sequence. In an example embodiment, actuating means 40 are used to provide a stimulus or stimuli causing a sensation in addition to the touch, for example a tactile sensation. In an embodiment, the touch toll is used not only to touch a certain point location, but to draw on the target area, for example to draw a form, a letter or a number.

At step 330 the imaging means are used to image the location touched, i.e. a still or video image is obtained of the area touched, the image showing the location in which the touch tool touches the target area. In an example embodiment, the obtained image, a still image or video image, is sent to the second apparatus 200 for processing and/or for displaying on the user interface unit. In an example embodiment, information on the location is sent to the second apparatus 200 for processing and/or for displaying on the user interface unit. Further, in an example embodiment, at step 330, the measurement means 30 are configured to measure the force and/or pressure with which the touch tool 10a,10b touches the location. In an example embodiment, the result of the measurement is sent to the second apparatus 200 for processing and/or for displaying on the user interface unit. The information on the location touched and/or the force or pressure applied is in an embodiment used to assess the cognitive capabilities of the subject, for example to test the sensory threshold. In an example embodiment, the test setup, i.e. the locations touched and/or the force or pressure applied is stored for later use and/or comparison.

At 340 the location at which the touch tool touches the target area is calculated from the image taken at step 330 and/or using information on the location received. In an embodiment, the location is stored into memory and/or displayed on the user interface unit. In a further example embodiment, the location is shown on the user interface unit of the second apparatus 200 only intermittently, or only on a further apparatus which apparatus the subject of the test does not see, and the user interface unit of the second apparatus subsequently shows a general image of the area, i.e. an image of the area without indication of the locations. In an embodiment, the test subject is asked at a later step to identify the location or locations for example for testing memory functions.

In an example embodiment, the steps 320 to 340 are repeated several times in order to provide a pattern or a sequence of touch locations on the area used for testing, for example in a case where the touch locations are to form a certain geometrical form on the area to be tested, which form the test subject should then recognize, or in a case where the memory and/or further cognitive functions of the test subject is to be tested.

At step 350 user input is received from the subject of the test. The subject is prompted to provide input via the user interface unit of the second apparatus 200. In an embodiment, the user input comprises the user indicating the location which was touched by the touch tool 10a,10b. In an example embodiment, the user input comprises the subject indicating a form that was formed by the touch locations, for example by drawing on the user interface means or selecting a form from a selection of forms displayed on the user interface means. In a further example embodiment, the user input comprises the subject indicating a sequence of touch locations. In an example embodiment, the speed with which the user input is provided by the subject forms a part of the user input. In a still further example embodiment the subject input comprises the user indicating the hardest touch. In an example embodiment, the steps 320 to 350 are repeated several times. In an example embodiment, information on the user input is sent to the first apparatus 100 the control means of which are configured to receive the information on the user input, for example in order to repeat the steps 320 to 350 adapted with the information on the user input.

At step 360, the result of the testing is calculated. In an example embodiment, the result is shown to the user, for example an operator of the apparatus, and/or to the subject of the testing on the user interface unit of the second apparatus 200. In a further example embodiment, the method, i.e. the testing, is repeated at regular or irregular intervals for the subject, and accordingly the progress or changes in functions that are tested is monitored as the results from the testing are compared over time. In an example embodiment, the testing is repeated at a later time with the same test setup in order to monitor progress or change in the performance to be tested. In an example embodiment, the results of the test are stored for comparison at a later time, for example also as an image or sequence of images such as video.

Some example use cases of the method and apparatus for human sensory testing are presented in the following. In a first use case the method and apparatus for human sensory testing is used to objectively test and record the performance of different types of sensing receptors located on the skin surface and/or tongue.

In a second use case, the method and apparatus for human sensory testing is used to test a subject's ability to attend to the touching, keep in working memory the sequence and type(s) of touch and to be able to identify the form the dots on the skin form which requires interaction between working and long-term memory and abstract thinking.

In a third use case, the method and apparatus for human sensory testing is used to study hand-eye co-ordination using a touchscreen based user interface..

In a fourth use case, the method and apparatus for human sensory testing is used to study various cognitive functions in humans, for example age-related effects, the effects of different diseases that affect memory and cognition as well as behavior, and to provide new solutions for rehabilitation and following the results of rehabilitation and other treatments.

In a fifth use case, the method and apparatus for human sensory testing is used to identify which areas of the brain do not function properly, using the fact that the human body representation on the sensorimotor cortex is well known. Accordingly, the method and apparatus for human sensory testing is used to objectively test and record the functions of different brain areas and identify e.g. how different types of brain traumas, e.g. stroke, contusion, Parkinson's diseases and dementias like Alzheimer's disease, affect the different parts of the sensing capabilities from the receptor to the sensorimotor cortex, other brain areas giving meaning to what has been sensed and how a person interprets the sensing in terms of type and meaning.

In a sixth use case, the method and apparatus for human sensory testing is used in figuring out possible problems in the sensing-cognition chain and how to design e.g. rehabilitation measures and follow their efficacy.

In a seventh use case, the method and apparatus for human sensory testing is used to examine if the ability of a person to detect the specific areas of the tongue that are touched is abnormal or missing or if the ability to remember the sequence of touching or identifying a form produced by the "touch dots" doesn't work., thus providing easy testing capability that can help in doing differential diagnostics of tongue sense related problems.

In an eighth use case, the method and apparatus for human sensory testing is used to test the different flavors, i.e. sweet, salty, sour, bitter and umami, in order to get a comprehensive understanding of the tongue-related functions.

In a ninth use case, the method and apparatus for human sensory testing is used as a game, providing a fun way to test one's own capabilities and also compete with others.

In a tenth use case, the method and apparatus for human sensory testing is used to train the sensing abilities and memory functions of the user, as well as spatial skills.

In an eleventh use case, the method and apparatus for human sensory testing is used to teach numbers, letters, simple mathematics and basic writing, i.e. used as a part of education in for healthy people but also for teaching people with learning disabilities.

Without in any way limiting the scope, interpretation, or application of the claims appearing below, a technical effect of one or more of the example embodiments disclosed herein is the provision of a standardized way of testing receptor sensing capabilities and the performance of the function of sensorimotor cortex and the more cognitive associative sensor and -motor cortices. Another technical effect of one or more of the example embodiments disclosed herein is the provision of an easy to use method and apparatus for sensory testing. Another technical effect of one or more of the example embodiments disclosed herein is the provision of a simple test linking the relevant parts of sensing to interpretation chain. Another technical effect of one or more of the example embodiments disclosed herein is a inconspicuous test for sensory functions.

Embodiments of the present invention may be implemented in software, hardware, application logic or a combination of software, hardware and application logic. The software, application logic and/or hardware may reside on the second apparatus 200 or the control means 50. If desired, part of the software, application logic and/or hardware may reside on the second apparatus 200 and/or the control means 50, part of the software, application logic and/or hardware may reside on an external device, and/or part of the software, application logic and/or hardware may reside on a cloud service. In an example embodiment, the application logic, software or an instruction set is maintained on any one of various conventional computer-readable media. In the context of this document, a "computer-readable medium" may be any non-transitory media or means that can contain, store, communicate, propagate or transport the instructions for use by or in connection with an instruction execution system, apparatus, or device, such as a computer, with one example being the second apparatus 200 described and depicted in Fig. 2. A computer-readable medium may comprise a computer-readable storage medium that may be any media or means that can contain or store the instructions for use by or in connection with an instruction execution system, apparatus, or device, such as a computer.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications which may be made without departing from the scope of the present invention as defined in the appended claims.

## Claims

1. An apparatus for human sensory testing, comprising
imaging means for imaging a target area;
at least one touch tool configured to touch a location on the target area; and
control means; wherein
the imaging means are configured to image the location touched on the target area; and wherein
the control means are configured to send the image and/or information on the location and to receive information regarding user input.

2. The apparatus of claim 1, further comprising measurement means configured to measure a force or pressure with which the at least one touch tool touches the location on the target area.

3. The apparatus of claim 1 or 2, further comprising actuating means for providing a stimulus or stimuli causing a sensation on the location on the target area which the at least one touch tool is touching.

4. The apparatus of claim 1, 2 or 3, comprising a plurality of touch tools, each touch tool having a different shape and/or surface texture.

5. A system for sensory testing comprising
a first apparatus according to any of the claims 1-4; and
a second apparatus connected with the first apparatus, and comprising a processor and a user interface unit.

6. The system of claim 5, wherein the user interface unit is configured to receive user input.

7. The system of claim 5 or 6, wherein the user interface unit comprises a touch display.

8. The system of claim 5, 6 or 7 wherein the second apparatus comprises a personal electronic device.

9. A method for human sensory testing, comprising
imaging a target area to be used for sensory testing with imaging means of a first apparatus;
touching a location on the target area with at least one touch tool of a first apparatus;
imaging the location touched on the target area and sending the image and/or information on the location to a second apparatus; and
calculating the location from the image of the touched location and/or using the information on the location.

10. The method of claim 9, further comprising measuring the force or pressure with which the at least one touch tool is touching the location on the target area.

11. The method of claim 9 or 10, further comprising providing a stimulus or stimuli causing a sensation on the location on the target area.

12. The method of claim 9, 10 or 11, further comprising receiving user input with a user input unit of the second apparatus.

13. The method of any of claims 9 to 12, wherein receiving user input comprises the user indicating the location on the target area from an image on the user interface unit.

14. The method of any of claims 9 to 13, wherein the target area comprises human tongue or an area of human skin.
